# EUROPÄISCHE PATENTSCHRIFT

(11) **EP 3 525 918 B1**
(45) Veröffentlichungstag und Bekanntmachung des Hinweises auf die Patenterteilung: **13.01.2021**
(21) Anmeldenummer: 17793585.5
(22) Anmeldetag: 13.10.2017
(51) Int. Cl.: B01D 53/62, B01D 53/86, C10L 3/10, C01B 3/24, C07C 1/04, C07C 1/12

(54) **VERFAHREN ZUR ABSCHEIDUNG UND IMMOBILISERUNG VON KOHLENDIOXID UND/ODER KOHLENMONOXID AUS EINEM ABGAS**
METHOD FOR SEPARATING OFF AND IMMOBILIZING CARBON DIOXIDE AND/OR CARBON MONOXIDE FROM AN EXHAUST GAS
PROCÉDÉ DE SÉPARATION ET D'IMMOBILISATION DE DIOXYDE DE CARBONE ET/OU DE MONOXYDE DE CARBONE D'UN GAZ D'ÉCHAPPEMENT

(30) Priorität: 13.10.2016 DE 102016219990
(43) Veröffentlichungstag der Anmeldung: 21.08.2019
(73) Patentinhaber: Fulde, Marek, 63071 Offenbach (DE)
(72) Erfinder: Fulde, Marek, 63071 Offenbach (DE)
(74) Vertreter: Römer, Michael Werner
(86) Internationale Anmeldenummer: PCT/EP2017/076201
(87) Internationale Veröffentlichungsnummer: WO 2018/069504

(56) Entgegenhaltungen:
- DE-A1-102007 037 672
- DE-A1-102012 007 136
- DE-A1-102012 214 907
- DE-A1-102013 020 511
- DE-A1-102013 112 205

## Beschreibung

Die Erfindung betrifft ein Verfahren zur Abscheidung und Immobilisierung beziehungsweise Sequestrierung von Treibhausgas-Effekt verursachenden Stoffen, wie Kohlendioxid und/oder Kohlenmonoxid aus Abgasen.

Derartige Verfahren sind dem Fachmann bekannt als "Carbon dioxide Capture and Storage" (CCS) Verfahren. Die Sequestrierung von Treibhausgasen umfasst die Abscheidung von Kohlendioxid aus industriellen Quellen und Kraftwerken, den Transport zum Lagerort und schließlich eine dauerhafte Lagerung von CO₂ mit der Isolierung von der Atmosphäre.

Die während der Stromerzeugung oder in industriellen Prozessen entstehenden Abgase sind üblicherweise reich an dem Treibhausgas (THG) Kohlendioxid. Kohlenmonoxid, dessen klimaschädliche Wirkung noch nicht bestätigt wurde, stellt ebenfalls potentiell eine Umweltgefahr dar. Neben Treibhausgasen bestehen die Abgase überwiegend aus Stickstoff sowie aus anderen Luftbestandteilen wie Argon oder Sauerstoff. Die Konzentration von Sauerstoff ist relativ gering (2 bis 6 Vol.-%), da er in der Verbrennung fast vollständig verbraucht wird. Prozessbedingt treten in industriellen Abgasen weitere Gaskomponenten wie z.B. Wasserstoff oder Kohlenmonoxid auf.

Zur Vermeidung der THG-Emissionen wird bei dem bekannten CCS-Verfahren CO₂ abgeschieden und anschließend gelagert. Bei der Anwendung dieses Verfahrens wird das zu behandelnde Abgas zuerst von Partikeln und Schwefelverbindungen befreit. Im darauffolgenden Schritt wird CO₂ aus dem Abgas abgetrennt, komprimiert, gegebenenfalls abtransportiert und unterirdisch gelagert.

Es ist in der Wissenschaft umstritten, ob unter diesen Bedingungen eine dauerhafte Bindung von CO₂ an Mineralien stattfindet oder mit erneuten Freisetzungen in die Atmosphäre zu rechnen ist. Weiterhin könnte die Verpressung von CO₂ unter hohem Druck zu Erdbeben führen. Die Vermischung und Lösung von Kohlendioxid mit Grundwasser könnte auch sogenannte "Kalt-Geysire" hervorrufen, die ebenfalls erneute Emission verursachen würden.

Ein anderer Aspekt der erschwerten Nutzung von CCS-Technologie liegt in fehlender Infrastruktur, die für den Transport von CO₂ erforderlich wäre. Dies liegt darin begründet, dass die Emissionsquellen wie Kraftwerke oder Zementwerke weit von geeigneten CO₂-Lagerorten liegen, was den Bau von geeigneten Rohrleitungen erforderlich machen würde.

Im Dokument DE 10 2013 112 205 A1 wird darauf hingewiesen, dass der durch thermische Zersetzung von Methan erzeugte Kohlenstoff in alten Kohlenflözen oder anderswo langfristig und problemlos eingelagert werden kann.

Dokument DE 10 2007 037 672 A1 beschreibt ein Verfahren zur Harmonisierung der Strom-Angebots/Verbrauchs-Verläufe durch Zwischenspeicher und Einbeziehung einer CO₂-Verwertung. Demnach wird aus dem CO₂-haltigen Abgas reines CO₂ gewonnen und CO₂ zwischengelagert. Ein Teil des gelagerten CO₂ wird zur Hydrierung zu Kohlenwasserstoffen benutzt, die wieder in Kraftwerken zur Stromerzeugung verbrannt werden. Das verbleibende Kohlendioxid wird endgelagert, was einem bekannten CCS-Verfahren entspricht. Die Spaltung von Kohlenwasserstoffen ist nicht vorgesehen. Eine Lagerung von Kohlenstoff in fester Form findet nicht statt.

In WO 2015/044407 wird ein Verfahren zur Speicherung von Strom aus erneuerbaren Quellen beschrieben. Dabei wird reiner Wasserstoff, der durch Elektrolyse von Wasser gewonnen wird, mit reinem CO₂ oder einem CO₂/CO/H₂-Gemisch zu Methan umgesetzt. Methan wird zwischengelagert und anschließend zu Kohlenstoff und Wasserstoff gespalten. Kohlenstoff wird zur Herstellung von CO₂ oder eines CO₂/CO/H₂-Gemisches wiederverwendet. Wasserstoff wird der energetischen Nutzung, beispielsweise zur Stromerzeugung zugeführt. Kohlendioxid wird nicht gelagert, der Kohlenstoff wird in dem Verfahren vollständig in einem Kreislauf geführt.

Eine Aufgabe der Erfindung kann darin gesehen werden, klimarelevante Abgase zu immobilisieren, wobei Kohlenstoff der Atmosphäre dauerhaft und sicher entzogen und dauerhaft gelagert wird.

### Offenbarung der Erfindung

Es wird ein Verfahren zur Abtrennung und Immobilisierung von Kohlendioxid und/oder Kohlenmonoxid aus einem Abgas vorgeschlagen. Das Abgas wird insbesondere erhalten durch das Verbrennen von fossilen Brennstoffen in einem Kraftwerk, als Nebenprodukt in einem industriellen Prozess oder als Fördergas bei der Förderung fossiler Brennstoffe. Das Verfahren umfasst die Schritte:
a) Einstellen eines für eine Methanisierungsreaktion geeigneten stöchiometrischen Verhältnisses von Kohlendioxid zu Wasserstoff und/oder von Kohlenmonoxid zu Wasserstoff, indem dem Abgas eine entsprechende Menge an Wasserstoff oder alternativ Kohlendioxid und/oder Kohlenmonoxid mit einem Zusatzgas zugeführt wird,
b) Durchführen einer katalytischen Reaktion, bei der als Edukte Kohlendioxid und/oder Kohlenmonoxid sowie Wasserstoff in Methan und Wasser umgesetzt werden,
c) Abtrennen des Methans aus dem Produkt der katalytischen Reaktion,
d) Spalten des Methans in Kohlenstoff und Wasserstoff, wobei der Kohlenstoff als Feststoff anfällt,
e) Auffangen des abgespaltenen Kohlenstoffs und
f) Deponieren bzw. Endlagern des aufgefangenen Kohlenstoffs.

Bei dem Abgas kann es sich beispielsweise um ein Abgas handeln, welches beim Verbrennen von fossilen Brennstoffen in einem Kraftwerk entsteht. Das Abgas kann beispielsweise auch ein Nebenprodukt sein, welches in einem industriellen Prozess entsteht oder das Abgas kann ein als Fördergas sein, welches bei der Förderung fossiler Brennstoffe anfällt. Diese Abgase, welche normalerweise in die Atmosphäre abgegeben werden, werden nach deren Entstehung aufgefangen und mit dem vorgeschlagenen Verfahren behandelt.

Das Abgas ist ein Gemisch aus mindestens zwei Komponenten, wobei das Abgas zumindest Kohlendioxid oder Kohlenmonoxid als eine Komponente umfasst. Des Weiteren umfasst das Abgas als weitere Komponenten mindestens ein Inertgas wie Stickstoff oder Argon, wobei das mindestens ein Inertgas nach der Abtrennung gemäß Schritt c) im Abgas verbleibt. Neben Inertgasen können im Abgas noch weitere Komponenten enthalten sein, die sich in der in Schritt b) durchgeführten katalytischen Reaktion neutral verhalten. Ein Beispiel für eine solche neutrale Komponente ist Wasserdampf. Die neutralen Komponenten können nach der Abtrennung des Methans im Abgas verbleiben. In einer bevorzugten Ausführungsform sind die Komponenten des Abgases ausgewählt aus Kohlendioxid, Kohlenmonoxid, Wasserstoff und Methan, wobei in diesem Fall durch das vorgeschlagene Verfahren sämtliche dieser Komponenten des Abgases vollständig immobilisiert werden.

Bei den Abgasen, welche als Treibhausgas-Effekt verursachende Stoffe insbesondere Kohlendioxid und/oder Kohlenmonoxid enthalten, kann es sich insbesondere um Abgase aus industriellen Herstellungsprozessen oder aus Verbrennungsprozessen sowie um Fördergase handeln. Fördergase fallen beispielsweise bei der Förderung von fossilen Energieträgern wie Kohle, Erdöl oder Erdgas an. Ein Beispiel für ein Fördergas ist Grubengas.
Den größten Anteil an den anfallenden Abgasen trägt die Herstellung von elektrischem Strom aus fossilen Brennstoffen bei.

Ebenso bedeutend als Quelle für klimarelevante Abgase sind industrielle Prozesse. Ein Beispiel für einen industriellen Herstellungsprozess ist die Zementproduktion, bei der die Emissionen von CO₂ größtenteils auf das Kalzinierungsverfahren zurückzuführen sind. Bei der Herstellung von Eisen und Stahl sowie hierfür benötigten Hilfsstoffen enthalten die Produktionsabgase neben CO₂ Kohlenmonoxid und Methan. Einige Abgase wie beispielsweise Gichtgas aus der Stahlproduktion oder Koksofengas enthalten neben CO₂ und CH₄ auch Kohlenmonoxid. Ob CO ebenfalls klimaschädliche Auswirkungen aufweist, ist noch nicht abschließend geklärt. Der unterstellte Global-Warming-Potential-Faktor (GWP-Faktor) von CO soll bei 3 kg CO₂-Äquivalent/kg Emission liegen. Klimarelevante Abgase können beispielsweise auch in Raffinerien auftreten.

Die industriellen Abgase enthalten üblicherweise neben den bereits erwähnten Kohlenstoffoxiden andere Stoffe, die bei der Behandlung des Abgases berücksichtigt werden müssen. Hierzu zählen unter anderem Methan, Wasserstoff, Wasserdampf und das Inertgas Stickstoff, wobei der Anteil an Stickstoff bis zu 97 Vol.-% betragen kann. Als weitere Abgasbestandteile können Verunreinigungen wie Schwefelwasserstoff, Quecksilber und/oder Schwermetale enthalten sein. Diese Verunreinigungen bedürfen einer Vorbehandlung, um vorgeschriebene Grenzwerte zu erreichen. Auch Sauerstoff kann in Konzentration von bis zu 6 Vol.-% im Abgas von Kraftwerken vorgefunden werden. Abgase, die während der Förderung von beispielsweise Erdgas entstehen, enthalten neben Methan CO₂ und Stickstoff mit relevanten Anteilen.

In dem ersten Schritt a) des Verfahrens wird dem Abgas Wasserstoff oder alternativ Kohlendioxid und/oder Kohlenmonoxid zugeführt, sodass das Mol-Verhältnis zwischen Wasserstoff und Kohlendioxid und/oder zwischen Kohlenmonoxid und Wasserstoff bevorzugt den stöchiometrischen Koeffizienten der Sabatier-Reaktion

CO₂+4 H₂ -> CH₄ + 2 H₂O (1)

oder der CO-Methanisierung

CO + 3 H₂ -> CH₄ + H₂O (2)

entspricht. Dabei wird bei einem Überschuss von Wasserstoff Kohlendioxid und/oder Kohlenmonoxid zugeführt und bei einem Defizit an Wasserstoff bzw. einem Überschuss an Kohlenoxiden Wasserstoff zugeführt.

Für das Einstellen des stöchiometrischen Verhältnisses werden bevorzugt die im Abgas enthaltenen Anteile an Kohlendioxid und/oder an Kohlenmonoxid gemessen. Sofern das Abgas Wasserstoff als eine Komponente enthält, wird darüber hinaus bevorzugt der Anteil an Wasserstoff im Abgas gemessen. Des Weiteren kann zusätzlich der Anteil an im Abgas enthaltenen Sauerstoff bestimmt werden. Um einen Verlust von Wasserstoff durch eine Reaktion von Wasserstoff mit im Abgas vorhandenem Sauerstoff ausgleichen zu können, kann zudem vorgesehen sein, die Menge an Wasserstoff im hergestellten Gemisch und/oder dem Anteil an Sauerstoff im Abgas zu bestimmen. Die Menge an zuzuführenden Gas wird dann anhand des zu behandelnden Abgasvolumens und den ermittelten Anteilen der einzelnen Komponenten in dem Abgas bestimmt.

Ist das Abgas reich an Kohlendioxid und/oder Kohlenmonoxid, so wird mit dem Zusatzgas Wasserstoff zugeführt. Ist das Abgas reich an Wasserstoff, so werden mit dem Zusatzgas Kohlendioxid und/oder Kohlenmonoxid zugeführt.

Der Wasserstoff für das Zusatzgas wird bevorzugt durch ein Elektrolyseverfahren, beispielsweise durch Hochtemperatur-Dampfelektrolyse hergestellt. Dabei wird bevorzugt die Abwärme der Abgase zur Herstellung von Wasserdampf und/oder zur Erzeugung von elektrischem Strom verwendet. Bevorzugt wird bei der Elektrolyse zur Herstellung des Wasserstoffs zumindest teilweise Strom aus erneuerbaren Quellen verwendet. Für die Elektrolyse kann insbesondere auch elektrischer Strom aus erneuerbaren Quellen benutzt werden, der derzeit im Stromnetz nicht abgenommen werden kann. Eine weitere bevorzugte Quelle für den Wasserstoff sind industrielle Prozesse wie die NaCI-Elektrolyse zur Gewinnung von Chlor, bei der Wasserstoff als Nebenprodukt anfällt.

Der bei der Elektrolyse von Wasser ebenfalls anfallende Sauerstoff kann in die Atmosphäre abgeben werden. In einer bevorzugten Ausführungsform des Verfahrens wird der erzeugte Sauerstoff einem Verbrennungsprozess zugeführt, bei dem das mit dem beschriebenen Verfahren zu behandelnde Abgas entsteht. Hierdurch wird die Verbrennung mit reinem Sauerstoff oder zumindest mit einem Gasgemisch mit einem gegenüber dem normalen Sauerstoffanteil in der Atmosphäre erhöhten Sauerstoffgehalt durchgeführt. Dies erhöht die Effizienz des Verbrennungsprozesses und reduziert den Anteil an in dem Abgas enthaltenen Inertgasen. Das zu behandelnde Volumen an Abgas wird hierdurch verringert. Das Durchführen von Verbrennungsprozessen unter Zugabe von reinem Sauerstoff ist im Stand der Technik als "Oxyfuel"-Verfahren bekannt.

Kohlendioxid für das Zusatzgas wird bevorzugt durch Verbrennen kohlenstoffhaltiger Brennstoffe wie beispielsweise Biomasse oder Abfälle hergestellt. Die dabei entstehende Wärme wird im Produktionsprozess und/oder zur Stromerzeugung benutzt. Bevorzugt handelt es sich bei dem Zusatzgas um ein entsprechendes weiteres Abgas, welches somit im Rahmen dieses Verfahrens ebenfalls behandelt wird. Die Einstellung des für CO-Methanisierung erforderlichen Stoffverhältnisses ist mittels Kohlenmonoxid ebenfalls möglich.

Bevorzugt wird das Abgas vor der Zuführung des Zusatzgases mit üblichen, bereits bekannten Verfahren von Staub, schwefelhaltigen Verbindungen und anderen Verunreinigungen wie Stickstoffoxide, Chlorwasserstoff, Fluorwasserstoff, Quecksilber, andere Metalle und andere organische oder anorganische Substanzen gereinigt.

In dem zweiten Schritt b) des Verfahrens wird das Gemisch aus dem Abgas und dem zugegebenen Zusatzgas einer katalytischen Methanisierungsreaktion zugeführt, wobei Kohlendioxid und Wasserstoff und/oder Kohlenmonoxid und Wasserstoff zu Methan und Wasser umgesetzt werden. Bevorzugt handelt es sich bei der katalytischen Methanisierungsreaktion um eine Sabatier-Reaktion und/oder um eine CO-Methanisierungsreaktion. Mit Ausnahme von gegebenenfalls im Abgas bereits enthaltenem Wasserstoff sind die weiteren im Abgas enthaltenen Komponenten an den Methanisierungsreaktionen nicht beteiligt. Die Reaktionen finden bevorzugt bei Temperaturen von 250°C bis 350°C statt.

Beide Reaktionen (1) und (2) sind exotherm. Die produzierte überschüssige Wärme kann im Rahmen des Verfahrens genutzt werden, beispielsweise um Wasserdampf für eine Dampfelektrolyse bereitzustellen.

Das in den Reaktionen (1) und (2) entstandene Methan wird im Schritt c) des Verfahrens von Wasserdampf und gegebenenfalls weiteren im Abgas enthaltenen Komponenten befreit. Bevorzugt erfolgt das Abtrennen des Methans physikalisch, insbesondere mittels Kondensation, Adsorption oder einer Trennung mit Membranen. Des Weiteren ist es bevorzugt, Wasserdampf durch ein Kondensationsverfahren abzutrennen.

Bevorzugt wird das abgetrennte Methan qualitätskonform als Erdgas eingestellt und ins öffentliche Erdgasnetz eingespeist. Alternativ kann das Methan in einem Tank zwischengelagert werden. Die weiteren im Abgas enthaltenen Komponenten sind im Wesentlichen inerte Gase wie Stickstoff sowie Wasserdampf und können gefahrlos in die Umgebung abgegeben werden. Das abgetrennte Methan kann in dem Erdgasnetz gelagert und/oder zu einem Ort transportiert werden, an dem Wasserstoff benötigt wird, oder zu einer Kohlenstoff-Deponiestätte transportiert werden.

Die Abgas-Entstehung und die Kohlenstoffdeponierung können an einem oder an mehreren räumlich getrennten Orten realisiert werden. Dies wird durch die Transportfähigkeit des in Methan umgewandelten Abgases durch das öffentliche Erdgasnetz ermöglicht.

In dem nachfolgenden Schritt d) des Verfahrens erfolgt eine Spaltung des Methans in Kohlenstoff und Wasserstoff. Wurde das Methan zuvor in das Erdgasnetz eingespeist, wird nun Methan dem Erdgasnetz entnommen und in einem Spaltungsprozess in seine Elemente gespalten. Die Spaltung ist endotherm, sodass eine externe Energiequelle hierfür benötigt wird. Die Spaltung folgt der Reaktion:

CH₄ -> C + 2 H₂ (3)

Bevorzugt wird für die Spaltung des Methans ein Pyrolyseverfahren eingesetzt. Die für die Durchführung der Reaktion (3) benötigte Energie wird bevorzugt in Form von Strom aus erneuerbaren Quellen bereitgestellt, der derzeit im Stromnetz nicht abgenommen werden kann, sodass kein fossiler Brennstoff verwendet wird. Alternativ kann ein Teil des gewonnenen Wasserstoffs für das Bereitstellen der für die Spaltung notwendigen Energie verwendet werden. Vorteilhaft ist die Spaltung an der Stelle vorzunehmen, an der Wasserstoff als Brennstoff, Kraftstoff für Brennstoffzellen-Fahrzeuge oder als Rohstoff für Anwendungen beispielsweise in der chemischen Industrie Anwendung findet. Der Kohlenstoff wird gesammelt und zu einer Deponiestätte transportiert.

Bevorzugt wird zumindest ein Teil des bei der Spaltung erzeugten Wasserstoffs als Ausgangsstoff in der chemischen Industrie, als Energieträger für die Erzeugung von elektrischem Strom und/oder Wärme oder als Kraftstoff für Fahrzeuge verwendet.

Der als Feststoff entstehende Kohlenstoff wird aufgefangen und dauerhaft eingelagert bzw. endgelagert. Auf diese Art und Weise werden die aus den Abgasen "extrahierten" Treibhausgase der Atmosphäre entzogen.

Dabei wird der als Feststoff erhaltene Kohlenstoff vor dem dauerhaften Lagern bzw. Deponieren mit einem Träger vermischt, um den Kohlenstoff endgültig zu immobilisieren und besonders sicher verwahren zu können. Als Träger kann beispielsweise Sand, Lehm, Kies, Bauschutt, Schlacken, Steine, Abfälle, insbesondere aus Industriedemontagen, oder eine Kombination mehrerer dieser Materialien eingesetzt werden.

Eine Lagerung des immobilisierten Kohlenstoffs erfolgt bevorzugt unterirdisch, beispielsweise in alten Bergwerken, insbesondere Kalibergwerken oder, Salzbergwerken. Der immobilisierte Kohlenstoff ist aber auch als Füllmaterial zum Verfüllen von Tagebauen, Abgrabungen, Kies-, Gips- oder Tongruben geeignet.

Um geologische Schäden zu vermeiden sowie um den Naturschutzverpflichtungen nachzugehen, werden stillgelegte Förderstätten umfangreichen Sanierungs- und Rekultivierungsmaßnahmen unterzogen. Dabei werden die Hohlräume der Untertagebergwerke sowie die Gruben selbst mit mineralischem Material aufgefüllt. Als geeignetes Mittel kommen Bauschutt, Schlacken, Steine, Abfälle aus Industriedemontagen sowie andere industrielle Abfälle, die eine ausreichende Festigkeit aufweisen, zum Einsatz. Bei alleiniger Verwendung von Kohlenstoff müsste dieser für das Verfüllen zuerst verpresst werden. Als vorteilhaft erweist sich das Vermengen des pulverförmigen Kohlenstoffs mit Mineralien oder Abfällen, da Kohlenstoff in die poröse Struktur der Mineralien eindringen und dort dauerhaft fixiert werden kann. Hiermit ist eine dauerhafte Immobilisierung des Kohlenstoffs möglich und somit ein Reinigungseffekt für die Atmosphäre erzielt.

### Vorteile der Erfindung

Mittels des erfindungsgemäß vorgeschlagenen Verfahrens können Abgase wie beispielsweise industrielle Abgase oder Fördergase von klimaschädlichen Stoffen wie CO₂ sowie CO befreit werden. In dem Abgas gegebenenfalls enthaltenes Methan wird dem Abgas dabei vorteilhafterweise ebenfalls entzogen. Zudem kann gegebenenfalls im Abgas enthaltener Wasserstoff, der andernfalls ungenutzt in die Atmosphäre entweichen würde, ebenfalls genutzt werden. Als Edukt für die Methanisierungsreaktion werden vorgereinigte Abgase eingesetzt, ohne dass es eine vorgeschaltete, vollständige Oxidierung von CH₄ oder CO zu CO₂ bedarf.

Die Umwandlung der klimaschädlichen Stoffe in Kohlenstoff in fester Form vereinfacht in erheblichem Maße die Sequestrierung, da ein Feststoff und kein Gas immobilisiert wird. Das Finden einer geeigneten Kohlenstoff-Deponie erscheint um vielfaches leichter als das Auffinden geeigneter Lagerkapazitäten für gasförmiges CO₂, wie sie für herkömmliche CCS-Verfahren erforderlich sind.

Das Deponieren von Kohlenstoff in fester Form eliminiert die Risiken der Wiederemission. Geologische Risiken, die mit Verpressen des gasförmigen Kohlendioxids unter hohen Druck einhergehen, entfallen.

Das Verfahren erweitert die Möglichkeit der Sequestrierung von Kohlenstoff auf andere Treibhausgase wie Kohlenmonoxid, die im CCS-Verfahren nicht berücksichtigt werden. Des Weiteren werden gegebenenfalls enthaltenes Methan sowie Wasserstoff ebenfalls sequestriert und verwertet. Die bekannten CCS-Verfahren beziehen sich ausschließlich auf die Behandlung von Kohlendioxid. Methan wird bei den bekannten CCS-Verfahren nicht behandelt, obgleich die klimaschädliche Wirkung von Methan die von CO₂ bei weitem übersteigt.

Das Verfahren bietet auch die Möglichkeit, den Strom aus erneuerbaren Quellen zu speichern und zu transportieren, ohne auf ein Stromnetz angewiesen zu sein. Hierzu wird das als Zwischenprodukt des Verfahrens entstehende Methan über ein vorhandenes Erdgasnetz transportiert. Die Herstellung von Wasserstoff, der als Brennstoff oder Kraftstoff die Verwendung finden kann, öffnet breite Möglichkeiten der Sektorenkopplung zwischen Strom, Wärme, Mobilität oder stofflicher Nutzung. Ebenso ermöglicht das hergestellte Methan einen Transport des Kohlenstoffs zu einer Deponiestätte unter Verwendung des vorhandenen Erdgasnetzes.

Die industriellen Abgase sind oft durch ein hohes energetisches Potential gekennzeichnet. Die Nutzung des Potentials insbesondere zur Herstellung von Wasserdampf und zum Betreiben einer Hochtemperatur-Wasserdampfelektrolyse trägt zu einer erhöhten Effektivität des erfindungsgemäßen Verfahrens bei.

Eine der Möglichkeiten zur Erhöhung der Effizienz von CCS ist das "Oxyfuel"-Verfahren, bei dem anstelle von Luft reiner Sauerstoff oder ein mit Sauerstoff angereichertes Gemisch für die Verbrennung benutzt wird. Vorteilhaft ist hier die Abwesenheit von Luftstickstoff oder zumindest eine Reduktion des Anteils an Luftstickstoff, was die Konzentration von CO₂ im Abgas erhöht und die Abscheidung von CO₂ begünstigt. Nachteilig ist die Notwendigkeit, reinen Sauerstoff mittels Luftzerlegung bereitstellen zu müssen.

Bei dem vorgeschlagenen Verfahren ist die Wasser- oder WasserdampfElektrolyse Bestandteil des Prozesses, sodass Sauerstoff als Nebenprodukt neben Wasserstoff hergestellt wird. Er kann für die stickstofffreie bzw. stickstoffreduzierte Verbrennung eingesetzt werden. Eine Luftzerlegungsanlage würde entfallen. Dies trägt wesentlich zur Erhöhung der Wirtschaftlichkeit der Methode bei. Die Erfindung wird durch die Beispiele und Ansprüche erläutert.

### Beispiele

### Ausführungsbeispiel 1: Aufbereitung von Abgas aus der Zementproduktion

Bei der Produktion von Zementklinker entstehen THG-reiche Abgase. Zum einen entsteht Kohlendioxid in Folge der Entwässerungsreaktion von Kalziumverbindungen. Da diese Reaktion stark endotherm ist, wird große Menge Energie benötigt, die durch Verbrennen von Kohlenstoff erzeugt wird.

Im Projekt ECRA CCS ("ECRA CCS Project - Report on Phase III"; European Cement Research Academy; Düsseldorf; März 2012) wurde die Möglichkeit untersucht, die CO₂-Emissionen durch Abtrennung und Speicherung von Kohlendioxid zu reduzieren. Der Verbrennungsprozess soll auf das Oxyfuel-Verfahren umgestellt werden, wobei die Abgase im Kreis gefahren werden sollten. Diese Maßnahme hat zum Ziel, die Temperatur zu stabilisieren und die Konzentration von CO₂ in Abgas zu erhöhen. Die CO₂-Konzentration im Kreislaufgas beträgt 75 Vol.-% bis 82 Vol.-%. Die restlichen Bestandsteile sind unter anderem Kohlenmonoxid, Wasser, Sauerstoff, Stickstoff, Argon sowie Chloride und Stickstoffoxide in sehr geringen Konzentrationen. Ein Teil des in Kreis geführten Abgases wird abgetrennt, entstaubt, entschwefelt und von Verunreinigungen wie Stickstoffoxiden, Chlorwasserstoff, Fluorwasserstoff, Quecksilber, anderen Metallen und anderen organischen oder anorganischen Substanzen gereinigt, wobei bei dem CCS-Verfahren gemäß dem Stand der Technik an dieser Stelle auch Kohlenmonoxid abgetrennt und nicht weiter behandelt wird. Nach der Wasserkondensation wird CO₂ kryotechnisch abgetrennt. Das Kohlendioxid wird anschließend komprimiert und wird zum unterirdischen Lagerort transportiert. Der für das Oxyfuel-Verfahren notwendige Sauerstoff wird in einer Luftzerlegungsanlage gewonnen.

Bei der Anwendung des erfindungsgemäßen Verfahrens wird das beschriebene ECRA-Verfahren modifiziert, indem das CO₂-haltige Abgas nach Entstaubung, Entschwefelung und Entfernung von Verunreinigungen auf 0,8 MPa komprimiert wird. Eine Befreiung von CO und Wasser ist im Gegensatz zu dem Verfahren des Stands der Technik nicht notwendig.

Das komprimierte CO₂ haltige Gas wird zur Einstellung des stöchiometrischen Verhältnisses von 1:4 für Kohlendioxid zu Wasserstoff und von 1:3 für Kohlenmonoxid zu Wasserstoff mit Wasserstoff gemischt.

Der Wasserstoff wird durch Hochtemperatur-Wasserdampfelektrolyse am Standort gewonnen. Die in der Zementherstellung anfallende Abwärme wird zur Unterstützung der Elektrolyse verwendet. Der für die Elektrolyse benötigte Strom kann aus erneuerbaren Quellen stammen. Der bei der Elektrolyse anfallende Sauerstoff wird für das Oxyfuel-Verfahren verwendet, sodass im Gegensatz zum Stand der Technik eine Luftzerlegungsanlage nicht benötigt wird.

Das Gasgemisch wird einem Methanisierungsreaktor zugeführt. Die Reaktion wird in Anwesenheit von Nickel-Katalysator durchgeführt. Vorteilhaft ist ein isothermes Regime. Die Reaktionswärme wird in der Zementherstellung oder für die Elektrolyse verwendet. Nach der Abtrennung des Methans, bei der das Methan insbesondere über eine Trocknung von dem im Gasgemisch enthaltenen Wasser getrennt wird, und einer Qualität-Einstellung wird das Methangas in ein öffentliches Erdgasnetz eingespeist.

Die verbleibenden Restgase wie Stickstoff oder Argon sowie eine sehr geringe Menge Wasserdampf werden in die Atmosphäre abgeben.

An einem anderen Ort, an dem Wasserstoff beispielsweise für eine stoffliche Anwendung benötigt wird, wird Methan (Erdgas) aus dem Erdgasnetz entnommen und einer Pyrolyse unterzogen. Dabei wird Methan in Kohlenstoff (Ruß) und Wasserstoff gespalten. Ein Teil des Wasserstoffs wird als Energiequelle für die endotherme Pyrolyse-Reaktion benutzt. Der andere Teil wird beispielsweise als Rohstoff für die chemische Industrie verwendet. Alternativ kann die Pyrolyse-Reaktion mit elektrischem Strom betrieben werden.

Kohlenstoff wird von gasförmigem Wasserstoff abgetrennt und gesammelt. Er wird zur dafür genehmigten Deponiestätte transportiert und dort dauerhaft und sicher aufbewahrt.

### Ausführungsbeispiel 2: Aufbereitung von Abgas aus einem Kraftwerk

Die Abgase aus der herkömmlichen Stromproduktion enthalten abhängig von den für die Befeuerung benutzten Kohlensorten 3 Vol.-% bis 15 Vol.-% Kohlendioxid. Da die Befeuerung mit Luft erfolgt, bilden Stickstoff und Wasser die restlichen Hauptkomponenten der Abgase. Der Betriebsmodus der Kraftwerke richtet sich nach der optimalen, energetischen Nutzung des Brennstoffs. Dies hat zu Folge, dass in Abgasen 3 bis 5 Vol.-% Sauerstoff vorhanden sind.

Die Brennstoffe und folglich die Abgase sind mit Verunreinigungen wie Schwefelverbindungen, Stickstoffoxiden, Kohlenmonoxid und nicht selten mit Schwermetallen belastet.

Um das erfindungsgemäße Verfahren für CO₂-Abscheidung anzuwenden, wird das Abgas abgekühlt. Im nächsten Schritt folgen eine Entstaubung und Entschwefelung sowie eine selektive, katalytische Reinigung von Verunreinigungen wie Stickstoffoxide, Chlorwasserstoff, Fluorwasserstoff, Quecksilber, andere Metalle und andere organische oder anorganische Substanzen.

Dem gereinigten Abgas wird Wasserstoff zugemischt, um das für die Methanisierung notwendige stöchiometrische Verhältnis zwischen Kohlendioxid und Wasserstoff von 1:4 einzustellen. Hierfür werden die Konzentrationen von Kohlendioxid und Wasserstoff kontinuierlich online gemessen. Der Verbrauch an H₂ ist häufig höher als es alleine für die Einstellung des stöchiometrischen Verhältnisses erforderlich wäre, da Wasserstoff spontan mit Sauerstoff in dem Abgas zu Wasser reagiert. Daher ist es bevorzugt, dass auch der im Abgas vorhandene Anteil an Sauerstoff ermittelt wird.

Die Abgase der Kraftwerke sind üblicherweise drucklos, sodass die anschließende Methanisierung bei atmosphärischem Druck abläuft. Die Reaktion wird an einem Nickel-Katalysator bei 300°C - 350°C durchgeführt. Anschließend werden die Abgase abgekühlt. Die Trennung von Methan erfolgt mit Hilfe eines adsorptiven Verfahrens. Der gegebenenfalls in dem Abgas enthaltene Sauerstoff wird aufgrund der Wasserstoffzugabe in Wasser umgewandelt. Zur Speicherung des Methans kann dieser in einem Tank gelagert oder in ein öffentliches Erdgasnetz eingespeist werden.

Die verbleibenden Restgase wie Stickstoff oder Argon sowie sehr geringe Menge von Wasserdampf werden in die Atmosphäre abgeben.

An dem Ort, an dem der Wasserstoff beispielsweise für eine stoffliche Anwendung benötigt wird, wird Methan (Erdgas) aus dem Erdgasnetz entnommen und einer Pyrolyse unterzogen. Dabei wird Methan in Kohlenstoff (Ruß) und Wasserstoff gespalten. Ein Teil des Wasserstoffs wird für die endotherme Pyrolyse-Reaktion als Energiequelle benutzt.

Der andere Teil wird als Rohstoff für chemische Produktion verwendet. Alternativ kann die Pyrolyse-Reaktion mit elektrischem Strom betrieben werden. Kohlenstoff wird von gasförmigem Wasserstoff abgetrennt und gesammelt. Er wird zur dafür genehmigten Deponiestätte transportiert und dort dauerhaft und sicher aufbewahrt.

### Ausführungsbeispiel 3: Oxyfuel-Verbrennung

Die Verbrennung von sowohl fossilen wie auch erneuerbaren Brennstoffen mit Luft stellt den Stand der Verbrennungstechnik dar. Bei üblichen Verbrennungsprozessen bestehen die Abgase nach einer Wasserdampfkondensation zu 75 Vol.-% - 97 Vol.-% aus Stickstoff.

Im bekannten Oxyfuel-Verfahren wird Verbrennungsluft durch ein sauerstoffreiches Gas ersetzt. Um die Verbrennungstemperatur steuern zu können, werden die Abgase im Kreis geführt. Die Zugabe von Sauerstoff liegt bei 5 Vol.-% bis 20 Vol.-% des Kreislaufstroms. In gleicher Größenordnung wird das Abgas, bestehend aus CO₂ und gegebenenfalls Wasserdampf aber auch aus Stickstoff, Schwefelverbindungen und anderen Verunreinigungen wie Stickstoffoxide, Chlorwasserstoff, Fluorwasserstoff, Quecksilber, andere Metalle und andere organische oder anorganische Substanzen, ausgeschleust. Der Wasserdampf wird in einem Wärmetauscher auskondensiert. Nach Entstaubung, Entschwefelung und Reinigung von Schadstoffen steht CO₂₋haltiges Gas zur Verfügung.

Der für die Oxyfuel-Verbrennung benötigte Sauerstoff wird in Luftzerlegungs-Anlagen gewonnen. Das Verfahren ist dadurch energieintensiv.

Beim erfindungsgemäßen Verfahren wird das CO₂-haltige Abgas nach Abkühlung entstaubt, entschwefelt und von Verunreinigungen gereinigt. Anschließend wird das Abgas mit Wasserstoff vermischt, sodass das Verhältnis CO₂ zu H₂ bei 1:4 liegt. Im nächsten Schritt wird das Gasgemisch einem Reaktor zugeführt, in dem es an einem Nickel-Katalysator ins Methan-Gas umgesetzt wird. Die Reaktion findet bei Temperatur von 300°C bis 350°C und Druck von 0,7 MPa bis 0,8 MPa statt. Die Reaktionswärme wird zur Wasserdampf-Herstellung benutzt. Das während der Methanisierung entstehende Wasser wird auskondensiert und das Methan wird von weiteren Komponenten wie beispielsweise Stickstoff und Argon abgetrennt. Das gewonnene Methan wird nach der Einstellung der entsprechenden Qualität ins öffentliche Erdgasnetz eingespeist.

Die verbleibenden Restgase wie Stickstoff oder Argon sowie sehr geringe Menge von Wasserdampf werden in die Atmosphäre abgeben.

Wird der für das Verfahren notwendige Wasserstoff in einem Elektrolyse-Prozess gewonnen, kann der bei der Methanisierung gewonnene Wasserdampf als Ausgangsstoff für die Wasserdampfelektrolyse verwendet werden. Die Abwärme aus dem Verbrennungsprozess wird aufgrund ihrer hohen Temperatur zur Produktion von Dampf benutzt, der in der Dampfelektrolyse Anwendung findet. Der neben H₂ in der Elektrolyse entstehende Sauerstoff kann als Sauerstoffquelle für den Oxyfuel-Prozess eingesetzt werden, sodass auf eine Luftzerlegungsanlage verzichtet werden kann.

Wie in den vorangegangenen Ausführungsbeispielen gezeigt, wird Methan (Erdgas) an der Stelle aus dem Erdgas-Netz entnommen, an dem Wasserstoff für beispielsweise stoffliche Anwendungen benötigt wird. Das Methangas wird in einer Pyrolyse-Reaktion in Wasserstoff und Kohlenstoff gespalten. Ein Teil des Wasserstoffs wird für die endotherme Pyrolyse-Reaktion als Energiequelle eingesetzt. Der andere Teil wird als Rohstoff für chemische Produktion verwendet. Alternativ kann die Pyrolyse-Reaktion mit elektrischem Strom betrieben werden.

Kohlenstoff wird von gasförmigem Wasserstoff abgetrennt und gesammelt. Er wird zur dafür genehmigten Deponiestätte transportiert und dort dauerhaft und sicher aufbewahrt.

### Ausführungsbeispiel 4: Abgase aus Erdölraffinerien

Die Molkonzentration von H₂ in Abgasen aus Erdölraffinerien variiert zwischen 5 Mol.-% und 90 Mol.-%. Ferner enthalten die Abgase CO₂ (bis 8 Mol-%), kleine Mengen Kohlenmonoxid CO (bis 2 Mol.-%) und Kohlenwasserstoffe, vorwiegend Methan CH₄. Dieses Ausführungsbeispiel ist beispielhaft für Abgase, bei denen der Anteil des enthaltenen Wasserstoffs dominierend ist.

Beispielhaft kann das Abgas aus 60 Vol.-% H₂, 30 Vol.-% CH₄, 8 Vol.-% CO₂ und 2 Vol.-% CO bestehen. Zum Einstellen der stöchiometrischen Verhältnisse CO₂:H₂ und CO:H₂ wird als Zusatzgas Abgas aus dem Dampferzeuger der Erdölraffinerie benutzt, das beispielsweise als wesentliche Komponenten 9 Vol.-% CO₂, 18 Vol.-% Wasserdampf und 73 Vol.-% Stickstoff enthält

In die sich anschließende katalytische Methanisierung wird somit nach Mischung der beiden Massenströme ein Eduktgemisch gemäß Tabelle 1 eingegeben.

**Tabelle 1**

| | |
|---|---|
| CH₄ | 30 mol |
| H₂ | 60 mol |
| CO | 2 mol |
| CO₂ | 12,5 mol (8 mol aus Raffinerieabgas und 5,5 mol aus Dampferzeugerabgas) |
| N₂ | 44 mol aus Dampferzeugerabgas |
| H₂O | 11 mol aus Dampferzeugerabgas |

Das als Zusatzgas verwendete Abgas aus dem Dampferzeuger kann als Verunreinigungen noch Schwefelverbindungen und Staub enthalten, die in der nächsten Verfahrensstufe entfernt werden. Das so aufbereitete CO₂/H₂O/N₂-Gemisch wird als Zusatzgas dem Wasserstoff haltigen Raffinerie-Abgas beigemischt. Dabei werden kontinuierlich die Massenströme und Konzentrationen im resultierenden Strom gemessen, sodass ein Mol-Verhältnis zwischen Wasserstoff und Kohlendioxid von 4:1 und Wasserstoff und Kohlenmonoxid von 3:1 eingestellt wird. Beim Beispiel-Abgas sind es 5,5 Volumenanteile von CO₂, die mit dem Zusatzgas beigemischt werden.

Das Gemisch wird auf die für die Methanisierungsreaktion vorteilhafte Temperatur von 300°C erwärmt. Die Methanisierungsreaktion kann bei atmosphärischem Druck durchgeführt werden. Die Umsetzung liegt bei 80% bei hoher Selektivität. Um die Umsetzungseffizienz zu erhöhen, ist es vorteilhaft, das Reaktionsgemisch auf Druck von 0,8 MPa zu komprimieren.

Das aufbereitete Gasgemisch wird dem Methanisierungsreaktor zugeführt. Eine vorgeschaltete Trennung des Stickstoffs ist nicht erforderlich, da das Inertgas die Selektivität und Ausbeute der Reaktion nicht beeinflusst. Die Reaktion wird in Anwesenheit von Nickel-Katalysator durchgeführt. Vorteilhaft ist ein isothermes Regime. Die Reaktionswärme wird zur Erzeugung von Wasserdampf genutzt.

Nach der Reaktion enthält das Gasgemisch 30 Vol.-% H₂O, 35 Vol.-% CH₄ und 35 Vol.-% N₂. Die weitere Aufarbeitung umfasst die Kondensation von Reaktionswasser und Abtrennung von Stickstoff, sodass schließlich das verbleibende Gasgemisch bestehend aus Methan und Reststickstoff die geforderte Qualität erreicht und in das öffentliche Gasnetz eingespeist wird.

Der verbleibende Stickstoff sowie eine sehr geringe Menge Wasserdampf werden in die Atmosphäre abgeben.

An einem anderen Ort, an dem Wasserstoff beispielsweise für eine stoffliche Anwendung benötigt wird, wird Methan (Erdgas) aus dem Erdgasnetz entnommen und einer Pyrolyse unterzogen. Dabei wird Methan in Kohlenstoff (Ruß) und Wasserstoff gespalten. Ein Teil des Wasserstoffs wird für die endotherme Pyrolyse-Reaktion als Energiequelle benutzt. Der andere Teil wird als Rohstoff für chemische Produktion verwendet.

Kohlenstoff wird von gasförmigem Wasserstoff abgetrennt und gesammelt. Er wird zur dafür genehmigten Deponiestätte transportiert und dort dauerhaft und sicher aufbewahrt.

### Kurze Beschreibung der Figuren

Ausführungsbeispiele der Erfindung sind in den Zeichnungen dargestellt und in der nachfolgenden Beschreibung näher erläutert.
Figur 1 zeigt eine schematische Darstellung eines ersten Ausführungsbeispiels des Verfahrens, bei dem das Abgas einen Überschuss an Kohlenoxiden aufweist und
Figur 2 zeigt eine schematische Darstellung eines zweiten Ausführungsbeispiels des Verfahrens, bei dem das Abgas einen Überschuss an Wasserstoff aufweist.

In der nachfolgenden Beschreibung der Ausführungsbeispiele der Erfindung werden gleiche oder ähnliche Komponenten und Elemente mit gleichen Bezugszeichen bezeichnet, wobei auf eine wiederholte Beschreibung dieser Komponenten oder Elemente in Einzelfällen verzichtet wird. Die Figuren stellen den Gegenstand der Erfindung nur schematisch dar.

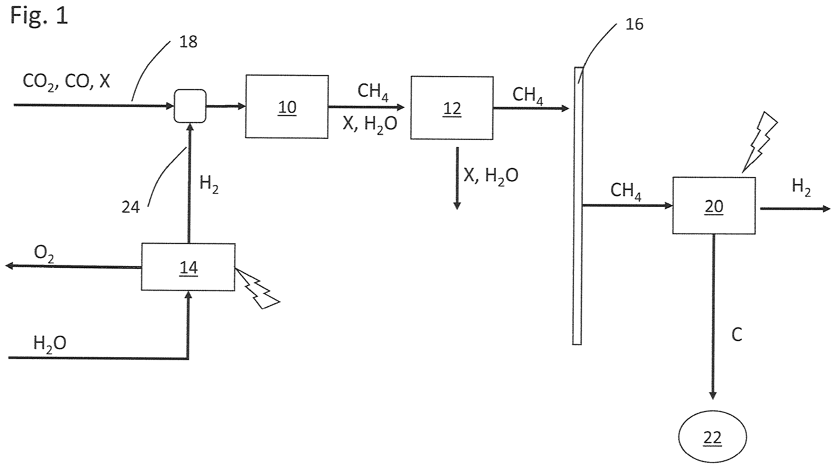

In der Figur 1 ist ein erstes Ausführungsbeispiel des Verfahrens dargestellt, bei dem ein an Kohlenstoffoxiden reiches Abgas 18, welches in dem dargestellten Beispiel CO₂, CO und mit "X" gekennzeichnete weitere Komponenten wie Stickstoff und Argon enthält, mit H₂ als Zusatzgas 24 vermischt wird, um ein für eine nachfolgende Methanisierungsreaktion optimales stöchiometrisches Verhältnis von CO₂ zu H₂ und CO zu H₂ einzustellen. Der Wasserstoff H₂ wird dabei über einen Elektrolyseur 14 aus Wasser H₂O unter Verwendung von elektrischer Energie hergestellt. Der bei der Elektrolyse ebenfalls anfallende Sauerstoff O₂ wird in dem dargestellten Beispiel in die Atmosphäre abgegeben. Alternativ kann der Sauerstoff O₂ auch einem Verbrennungsprozess zugeführt werden, bei dem das Abgas 18 entsteht.

Das mit dem Wasserstoff H₂ vermischte Abgas 18 wird einem Reaktor 10 zugeführt, in dem eine katalytische Methanisierungsreaktion durchgeführt wird. Dabei reagieren Kohlendioxid CO₂ und Wasserstoff H₂ sowie Kohlenmonoxid CO und Wasserstoff H₂ zu Methan CH₄ und Wasser H₂O. In einer Trennvorrichtung 12 werden das bei der Methanisierung erzeugte Wasser H₂O sowie die weiteren Komponenten X vom Methan CH₄ abgetrennt. Das Methan CH₄ wird anschließend in ein Erdgasnetz 16 eingespeist.

An dem Ort, an dem eine Deponierung von Ruß C erfolgen kann, wird das Methan CH₄ dem Erdgasnetz 16 entnommen und einem Methan-Spalter 20 zugeführt. In dem Methan-Spalter 20 wird das Methan CH₄ über eine Pyrolyse in Ruß C und Wasserstoff H₂ aufgespalten. Der Wasserstoff H₂ steht anschließend für eine weitere Verwertung zur Verfügung, beispielsweise als Ausgangsstoff für die chemische Industrie oder als Energieträger.

Der bei der Spaltung des Methans CH₄ in Form von Ruß C anfallende Kohlenstoff wird in einer Kohlenstoffdeponie 22 eingelagert und hierdurch dauerhaft der Atmosphäre entzogen.

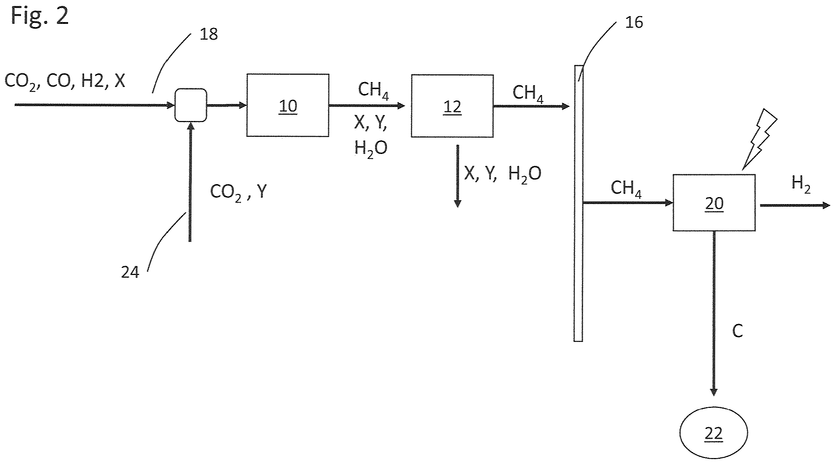

Die Figur 2 zeigt eine schematische Darstellung eines zweiten Ausführungsbeispiels des Verfahrens. Ein wasserstoffreiches Abgas 18, welches in dem dargestellten Beispiel CO₂, CO, H₂ und mit "X" gekennzeichnete weitere Komponenten enthält, wird mit einem Zusatzgas 24, welches CO₂ enthält, vermischt, um ein für eine nachfolgende Methanisierungsreaktion optimales stöchiometrisches Verhältnis von CO₂ zu H₂ und CO zu H₂ einzustellen.

Das Zusatzgas 24 kann mit "Y" gekennzeichnete weitere Komponenten enthalten wie beispielsweise H₂O oder N₂.

Das resultierende Eduktgemisch wird einem Reaktor 10 zugeführt, in dem eine katalytische Methanisierungsreaktion durchgeführt wird. Dabei reagieren Kohlendioxid CO₂ und Wasserstoff H₂ sowie Kohlenmonoxid CO und Wasserstoff H₂ zu Methan CH₄ und Wasser H₂O. In einer Trennvorrichtung 12 werden das bei der Methanisierung erzeugte Wasser H₂O sowie die gegebenenfalls enthaltenen weiteren Komponenten "X" und "Y" vom Methan CH₄ abgetrennt. Das Methan CH₄ wird anschließend in ein Erdgasnetz 16 eingespeist.

An dem Ort, an dem eine Deponierung von Ruß C erfolgen kann, wird das Methan CH₄ dem Erdgasnetz 16 entnommen und einem Methan-Spalter 20 zugeführt. In dem Methan-Spalter 20 wird das Methan CH₄ über eine Pyrolyse in Ruß C und Wasserstoff H₂ aufgespalten. Der Wasserstoff H₂ steht anschließend für eine weitere Verwertung zur Verfügung, beispielsweise als Ausgangsstoff für die chemische Industrie oder als Energieträger.

Der bei der Spaltung des Methans CH₄ in Form von Ruß C anfallende Kohlenstoff wird in einer Kohlenstoffdeponie 22 eingelagert und hierdurch dauerhaft der Atmosphäre entzogen.

Die Erfindung ist nicht auf die hier beschriebenen Ausführungsbeispiele und die darin hervorgehobenen Aspekte beschränkt. Vielmehr ist innerhalb des durch die Ansprüche angegebenen Bereichs eine Vielzahl von Abwandlungen möglich, die im Rahmen fachmännischen Handelns liegen.

### Bezugszeichenliste

- 10: Reaktor
- 12: Trennvorrichtung
- 14: Elektrolyseur
- 16: Erdgasnetz
- 18: Abgas
- 20: Methanspalter
- 22: Kohlenstoffdeponie
- 24: Zusatzgas

## Patentansprüche

1. Verfahren zur Abscheidung und Immobilisierung von Kohlendioxid und/oder Kohlenmonoxid aus einem Abgas (18), wobei das Abgas (18) erhalten wird durch das Verbrennen von fossilen Brennstoffen in einem Kraftwerk, als Nebenprodukt in einem industriellen Prozess oder als Fördergas bei der Förderung fossiler Brennstoffe, umfassend die Schritte:
a) Einstellen eines für eine Methanisierungsreaktion geeigneten stöchiometrischen Verhältnisses von Kohlendioxid zu Wasserstoff und/oder von Kohlenmonoxid zu Wasserstoff, indem dem Abgas (18) eine entsprechende Menge an Wasserstoff oder alternativ Kohlendioxid und/oder Kohlenmonoxid mit einem Zusatzgas (24) zugeführt wird,
b) Durchführen einer katalytischen Reaktion, bei der als Edukte Kohlendioxid und/oder Kohlenmonoxid sowie Wasserstoff in Methan und Wasser umgesetzt werden,
c) Abtrennen des Methans aus dem Produkt der katalytischen Reaktion,
d) Spalten des Methans in Kohlenstoff und Wasserstoff, wobei der Kohlenstoff als Feststoff anfällt,
e) Auffangen des abgespaltenen Kohlenstoffs und
f) Deponieren des aufgefangenen Kohlenstoffs,
wobei das Abgas (18) ein Gemisch aus mindestens zwei Komponenten ist, wobei das Abgas (18) zumindest Kohlendioxid oder Kohlenmonoxid als eine Komponente umfasst und das Abgas (18) als eine weitere Komponente mindestens ein Inertgas umfasst, welches nach der Abtrennung gemäß Schritt c) im Abgas (18) verbleibt und wobei der als Feststoff erhaltene Kohlenstoff vor dem Deponieren gemäß Schritt f) mit einem Träger vermischt wird, um den Kohlenstoff endgültig zu immobilisieren.

2. Verfahren gemäß Anspruch 1, **dadurch gekennzeichnet, dass** zum Einstellen des stöchiometrischen Verhältnisses die im Abgas (18) enthaltenen Anteile an Kohlendioxid und/oder an Kohlenmonoxid gemessen werden, wobei gegebenenfalls zusätzlich der im Abgas (18) enthaltene Anteil an Wasserstoff gemessen wird.

3. Verfahren gemäß Anspruch 1 oder 2, **dadurch gekennzeichnet, dass** im Schritt a) ein stöchiometrisches Verhältnis von 4 Wasserstoffmolekülen auf 1 Molekül Kohlendioxid und/oder 3 Wasserstoffmolekülen auf 1 Molekül Kohlenmonoxid im Abgas (18) eingestellt wird, wobei bei einem Überschuss an Wasserstoff im Abgas (18) ein Kohlendioxid und/oder Kohlenmonoxid enthaltendes Zusatzgas (24) verwendet wird und bei einem Defizit an Wasserstoff ein Wasserstoff enthaltendes Zusatzgas (24) verwendet wird.

4. Verfahren gemäß einem der Ansprüche 1 bis 3, **dadurch gekennzeichnet, dass** die Abtrennung des Methans physikalisch erfolgt, insbesondere mittels Kondensation, Adsorption oder Trennung mit Membranen.

5. Verfahren gemäß einem der Ansprüche 1 bis 4, **dadurch gekennzeichnet, dass** die Spaltung des Methans über ein Pyrolyseverfahren erfolgt.

6. Verfahren gemäß einem der Ansprüche 1 bis 5, **dadurch gekennzeichnet, dass** bei der Spaltung des Methans Wasserstoff erzeugt wird und dass die für die Spaltung benötigte Energie durch teilweises Verwerten des Wasserstoffs bereitgestellt wird.

7. Verfahren gemäß einem der Ansprüche 1 bis 6, **dadurch gekennzeichnet, dass** bei der Spaltung des Methans Wasserstoff erzeugt wird und dass zumindest ein Teil des Wasserstoffs als Ausgangsstoff in der chemischen Industrie oder als Energieträger für die Erzeugung von elektrischem Strom, Wärme oder als Kraftstoff für Fahrzeuge verwendet wird.

8. Verfahren gemäß einem der Ansprüche 1 bis 7, **dadurch gekennzeichnet, dass** das in Schritt c) abgetrennte Methan in ein Gasnetz eingespeist wird und für die Ausführung des Schritts d) des Verfahrens Methan aus dem Gasnetz entnommen wird.

9. Verfahren gemäß einem der Ansprüche 1 bis 8, **dadurch gekennzeichnet, dass** das Abgas (18) in einer Vorbehandlung entschwefelt, von anderen Verunreinigungen gereinigt wird und/oder entstaubt wird.

10. Verfahren gemäß einem der Ansprüche 1 bis 9, **dadurch gekennzeichnet, dass** der in Schritt a) zugeführte Wasserstoff über ein Elektrolyseverfahren gewonnen wird, wobei der ebenfalls anfallende Sauerstoff einem Verbrennungsprozess zugeführt wird, in dem das mit dem Verfahren behandelte Abgas entsteht.

## Claims

1. Process for separation and immobilization of carbon dioxide and/or carbon monoxide from an offgas (18), wherein the offgas (18) is obtained by combustion of fossil fuels in a power plant, as a byproduct in an industrial process or as an extraction gas in the extraction of fossil fuels, comprising the steps of:
a) establishing a stoichiometric ratio of carbon dioxide to hydrogen and/or of carbon monoxide and hydrogen which is suitable for a methanization reaction by supplying the offgas (18) with a corresponding amount of hydrogen or alternatively carbon dioxide and/or carbon monoxide by means of an additive gas (24),
b) performing a catalytic reaction in which the reactants carbon dioxide and/or carbon monoxide and also hydrogen are converted into methane and water,
c) separating the methane from the product of the catalytic reaction,
d) cracking the methane into carbon and hydrogen, wherein the carbon is generated as a solid,
e) collecting the cracked carbon and
f) landfilling the collected carbon
wherein the offgas (18) is a mixture of at least two components, wherein the offgas (18) comprises at least carbon dioxide or carbon monoxide as one component and the offgas (18) comprises at least one inert gas as further component which remains in the offgas (18) after the separation according to step c), and wherein the carbon obtained as a solid is mixed with a carrier before the landfilling of step f) in order to ensure permanent immobilization of the carbon.

2. The process as claimed in claim 1, **characterized in that** to establish the stoichiometric ratio the proportions of carbon dioxide and/or of carbon monoxide present in the offgas (18) are measured, wherein the proportion of hydrogen present in the offgas (18) is optionally also measured.

3. The process as claimed in claim 1 or 2, **characterized in that** in step a) a stoichiometric ratio of 4 hydrogen molecules to 1 molecule of carbon dioxide and/or 3 hydrogen molecules to 1 molecule of carbon monoxide is established in the offgas (18), wherein in the case of an excess of hydrogen in the offgas (18) an additive gas (24) containing carbon dioxide and/or carbon monoxide is used and in the case of a deficit of hydrogen an additive gas (24) containing hydrogen is used.

4. The process as claimed in any of claims 1 to 3, **characterized in that** the separation of the methane is effected by physical means, in particular by condensation, adsorption or separation with membranes.

5. The process as claimed in any of claims 1 to 4, **characterized in that** the cracking of the methane is effected by means of a pyrolysis process.

6. The process as claimed in any of claims 1 to 5, **characterized in that** the cracking of the methane produces hydrogen and **in that** the energy required for the cracking is provided by partial utilization of the hydrogen.

7. The process as claimed in any of claims 1 to 6, **characterized in that** the cracking of the methane produces hydrogen and **in that** at least a portion of the hydrogen is used as a starting material in the chemical industry or as an energy carrier for the generation of electricity, heat or as a fuel for vehicles.

8. The process as claimed in any of claims 1 to 7, **characterized in that** the methane separated in step c) is fed into a gas network and to perform step d) of the process methane is withdrawn from the gas network.

9. The process as claimed in any of claims 1 to 8, **characterized in that** the offgas (18) is desulfurized, purified of other impurities and/or dedusted in a pretreatment.

10. The process as claimed in any of claims 1 to 9, **characterized in that** the hydrogen supplied in step a) is produced by means of an electrolysis operation, wherein the oxygen likewise generated is supplied to a combustion operation in which the offgas treated with the process is formed.

## Revendications

1. Procédé pour la séparation et l'immobilisation de dioxyde de carbone et/ou de monoxyde de carbone d'un gaz d'échappement (18), le gaz d'échappement (18) étant obtenu par la combustion de combustibles fossiles dans une centrale, en tant que sous-produit dans un processus industriel ou en tant que gaz d'entraînement dans l'entraînement de combustibles fossiles, comprenant les étapes de :
a) ajustement d'un rapport stœchiométrique de dioxyde de carbone sur hydrogène et/ou de monoxyde de carbone sur hydrogène, approprié pour une réaction de méthanisation, en fournissant au gaz d'échappement (18) une quantité correspondante d'hydrogène ou alors de dioxyde de carbone et/ou de monoxyde de carbone comportant un gaz d'apport (24),
b) réalisation d'une réaction catalytique, dans laquelle du dioxyde de carbone et/ou du monoxyde de carbone ainsi que de l'hydrogène, en tant qu'éduits, sont transformés en méthane et en eau,
c) séparation du méthane du produit de la réaction catalytique,
d) fragmentation du méthane en carbone et en hydrogène, le carbone étant produit en tant que solide,
e) collecte du carbone fragmenté et
f) entreposement du carbone collecté,
le gaz d'échappement (18) étant un mélange d'au moins deux composants,
le gaz d'échappement (18) comprenant, en tant que composant, au moins du dioxyde de carbone ou du monoxyde de carbone et le gaz d'échappement (18) comprenant, en tant que composant supplémentaire, au moins un gaz inerte qui reste dans le gaz d'échappement (18) après la séparation selon l'étape c) et le carbone obtenu en tant que solide étant mélangé avec un support avant l'entreposement selon l'étape f), afin d'immobiliser définitivement le carbone.

2. Procédé selon la revendication 1, **caractérisé en ce que** pour l'ajustement du rapport stœchiométrique, les proportions de dioxyde de carbone et/ou de monoxyde de carbone contenues dans le gaz d'échappement (18) sont mesurées, éventuellement de plus la proportion d'hydrogène contenue dans le gaz d'échappement (18) étant mesurée.

3. Procédé selon la revendication 1 ou 2, **caractérisé en ce que** dans l'étape a), un rapport stoechiométrique de 4 molécules d'hydrogène pour 1 molécule de dioxyde de carbone et/ou 3 molécules d'hydrogène pour 1 molécule de monoxyde de carbone dans le gaz d'échappement (18) est ajusté, dans lequel lors d'un excès d'hydrogène dans le gaz d'échappement (18), un gaz d'apport (24) contenant du dioxyde de carbone et/ou du monoxyde de carbone est utilisé et lors d'un défaut en hydrogène, un gaz d'apport (24) contenant de l'hydrogène est utilisé.

4. Procédé selon l'une quelconque des revendications 1 à 3, **caractérisé en ce que** la séparation du méthane est réalisée de manière physique, en particulier au moyen d'une condensation, d'une adsorption ou d'une séparation avec des membranes.

5. Procédé selon l'une quelconque des revendications 1 à 4, **caractérisé en ce que** la fragmentation du méthane est réalisée par un procédé de pyrolyse.

6. Procédé selon l'une quelconque des revendications 1 à 5, **caractérisé en ce que** lors de la fragmentation du méthane, de l'hydrogène est produit et **en ce que** l'énergie nécessaire pour la fragmentation est mise à disposition par la valorisation partielle de l'hydrogène.

7. Procédé selon l'une quelconque des revendications 1 à 6, **caractérisé en ce que** lors de la fragmentation du méthane, de l'hydrogène est produit et **en ce qu'**au moins une partie de l'hydrogène est utilisée comme produit de départ dans l'industrie chimique ou en tant que source d'énergie pour la production de courant électrique, de chaleur ou en tant que combustible pour véhicules.

8. Procédé selon l'une quelconque des revendications 1 à 7, **caractérisé en ce que** le méthane séparé dans l'étape c) est alimenté dans un réseau gazier et pour la mise en œuvre de l'étape d) du procédé, du méthane est prélevé du réseau gazier.

9. Procédé selon l'une quelconque des revendications 1 à 8, **caractérisé en ce que** le gaz d'échappement (18) est, dans un prétraitement, désulfuré, purifié d'autres impuretés et/ou dépoussiéré.

10. Procédé selon l'une quelconque des revendications 1 à 9, **caractérisé en ce que** l'hydrogène fourni dans l'étape a) est obtenu par un procédé d'électrolyse, l'oxygène également produit étant fourni à un processus de combustion dans lequel le gaz d'échappement traité par le procédé est produit.
